# EUROPEAN PATENT APPLICATION

(11) **EP 4 477 218 A1**
(43) Date of publication of application: **18.12.2024**
(21) Application number: 23382592.6
(22) Date of filing: 15.06.2023
(51) Int. Cl.: A61K 31/195, A61K 31/404, A61K 31/41, A61K 31/425, A61K 31/496, A61K 31/5513, A61K 38/03, A61P 1/08, A61P 27/00

(54) **A CHOLECYSTOKININ (CCK) ANTAGONIST FOR USE IN THE PREVENTION AND/OR TREATMENT OF A VESTIBULAR DISEASE OR DISORDER**

(71) Applicant: Universitat Autònoma de Barcelona, 08193 Bellaterra (Cerdanyola del Vallès) (ES)
(72) Inventor: QUINTANA ROMERO, Albert, 08205 Sabadell (ES); SÁNCHEZ BENITO, Laura, 08290 Cerdanyola del Vallès (ES); MACHUCA MÁRQUEZ, Pablo, 29013 Málaga (ES); SANZ IGLESIAS, Elisenda, 08205 SSabadell (ES)

(57) **Abstract**

The present invention relates to a cholecystokinin (CCK) antagonist for use in the prevention and/or treatment of a vestibular disease or disorder selected from motion sickness, Meniere's disease, vestibular migraine, disembarkment sickness, labyrinthitis, vestibular neuronitis, benign paroxysmal positional vertigo, labyrinthine infarction and nausea.

## Description

### Field of the invention

The present invention relates to vestibular diseases or disorders. In particular, the present invention relates to a cholecystokinin (CCK) antagonist for use in the prevention and/or treatment of a vestibular disease or disorder.

### Background

US 9,167,998 mentions that disorders of the vestibular system present important clinical problems. Dizziness, a term which can embrace imbalance, vertigo, and light-headedness, affects more than 20% of the working age population, bedside tests suggest vestibular disorders in 35% of those over 40, and the prevalence is greater than 60% in the elderly. Using a more strict definition of vertigo, a prevalence of 7.4% was suggested, 80% of who seek medical consultation. Disorders of balance can be classified into one of two major categories: central vestibular disorders (i.e., disorders of the brain and central nervous system) and peripheral vestibular disorders (disease of the inner ear and eighth cranial nerve).

The main vestibular disorder to be considered herein is motion sickness (MS). Being described by Hippocrates over 2000 years ago, MS affects millions of individuals (36). The CNS is thought to compute an MS-triggering sensory conflict signal analogously to "toxic shock", eliciting malaise and nausea (8, 40, 52, 63). Thus, in an evolutionary context, MS responses could represent a proxy for toxicity-induced balance mismatches. Hence, vomiting observed in humans would be a response to evacuate toxic substances, appetite suppression to avoid additional toxic ingestion, hypolocomotion and hypothermia to minimize the metabolism, and CTA to avoid consumption of toxins in the future. However, the neurobiological underpinnings of MS have remained elusive.

Motion sickness (MS) is an unpleasant autonomic physiological alteration that occurs in healthy individuals undergoing passive or even illusory motion. MS signs and symptoms include pallor, cold sweating, yawning, retching and vomiting, vertigo, anorexia, drowsiness, and even severe pain (9, 23, 36, 39, 49). MS is highly conserved among species (24, 39, 42, 63, 65). Thus, while still debated (31), it has been posited that MS may be the byproduct of an evolutionary mechanism acting as an early toxin-ingestion warning system leading to a metabolic expense reduction (hypothermia and drowsiness), expulsion of the toxin (vomiting) and future avoidance (taste aversion) of the ingested substance (63).

It is widely accepted that MS arises from conflicts between actual visual/vestibular sensory inputs and the expected motion and body position information based on past memories (10, 45, 52). Accordingly, the necessity of a functional vestibular system for the development of MS was identified long ago (28, 29). Movement-related information processed by the vestibular organ in the inner ear is relayed directly to the medullo-pontine vestibular nuclei (VN). Compelling evidence demonstrates that VN neurons are particularly crucial in MS neurobiological regulation by showing that activation of VN neurons through provocative motion reproduced MS-like autonomic alterations in rats and mice (1, 38, 69). In addition, diseases caused by VN affectation are associated to autonomic dysregulation such as vertigo, nausea, and vomiting (18).

The role of the VN in the control of the body orientation system, mainly by means of vestibular reflexes at the ocular, head, neck, and spinal levels, has been amply described (3). Furthermore, vestibular outputs have been shown to modulate blood pressure with posture change (67, 68). However, how vestibular function governs motion-induced changes in other MS-mediated responses, such as hypolocomotion, appetite suppression, loss of body temperature, or acquisition of a conditioned taste aversion (CTA) (1, 13, 21, 36, 38, 39, 40, 42), is still unknown. In this regard, mapping of neuronal activity after provocative motion has identified potential neural substrates for vestibular-induced physiological responses, such as the Nucleus of the Solitary Tract (NTS), paraventricular nucleus of the hypothalamus (PVN), parabrachial nucleus (PBN), central amygdala (CeA), dorsal raphe nucleus, locus coeruleus and area postrema (1, 38). Among them, both the NTS and PBN are known to process visceral sensory input in the brainstem (46) and receive direct connections from the VN (4, 5, 34).

Excitatory neurons are the main projection-neuron type in the VN (19) and have been suggested to participate in autonomic responses after hypergravity (2) and postural imbalance (37). Furthermore, provocative-motion stimuli activate glutamatergic vesicular glutamate transporter 2 (VGLUT2)-expressing VN neurons that in turn project to downstream nuclei such as the PBN (14, 22, 26). Thus, we hypothesized that genetically defined, glutamatergic neuronal (sub)populations and circuit the VN are sufficient and/or necessary to develop MS-induced autonomic regulation and/or aversive learning. To that end, we used cell-type-specific transcriptomics combined with optogenetic and chemogenetic approaches to identify the underlying vestibular circuitry of MS-induced responses.

Accordingly, there is the need to find a suitable compound for the prevention and/or treatment of a vestibular disease or disorder, in particular for the prevention and/or treatment of motion sickness.

### Summary of the invention

In a first aspect the present invention relates to a cholecystokinin (CCK) antagonist for use in the prevention and/or treatment of a vestibular disease or disorder selected from motion sickness, Meniere's disease, vestibular migraine, disembarkment sickness, labyrinthitis, vestibular neuronitis, benign paroxysmal positional vertigo, labyrinthine infarction and nausea.

### Brief description of the drawings

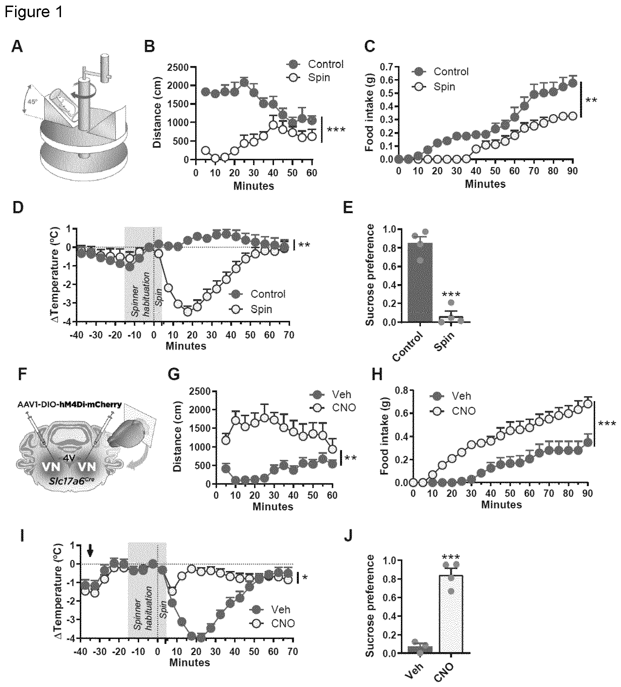

**Figure 1****. VGLUT2^{VN} neuron inactivation blocks rotation-induced kinetosis in mice.** (A) Mice were subjected to a rotational stimulus using a custom-made rotary device. Perpendicular distance from the mouse head to the axis of rotation was 5 cm. Mouse body inclination angle was 45°. (B) Traveled distance during a 60-min open-field test after spin or control stimulation (n=6; Two-way ANOVA, P <0.001). (C) Cumulative food intake after spin or control stimulation (n=6; Two-way ANOVA, P<0.01). Mice were food deprived for 24 h prior to the test. (D) Body temperature difference (ΔT) after spin or control stimulation (n=5; Two-way ANOVA, P<0.01). (E) Conditioned taste aversion (CTA) response in mice exposed to a two-bottle-based test pairing a 5% sucrose solution to rotational stimulus (n=4; t-test, P-value<0.001). (F) *Slc17a6^{Cre}* mice were bilaterally injected in the vestibular nuclei (VN) with AAV1-DIO-hM4Di mCherry (Vglut2^{VN}:hM4Di mice) to inhibit glutamatergic neurons upon CNO administration. (G) Total distance traveled during a 60-min open-field session after spin stimulation in CNO- or vehicle-injected Vglut2^{VN}:hM4Di mice (n=10; Two-way ANOVA, *P*<0.01). (H) Cumulative food intake after spin stimulation in CNO- or vehicle-injected Vglut2^{VN}:hM4Di mice (n=7; Two-way ANOVA, P<0.001). Mice were food deprived for 24 h prior to the test. (I) Core body temperature difference (ΔT) after spin stimulation in CNO- or vehicle-injected Vglut2^{VN}:hM4Di mice (n=4; Two-way ANOVA, P<0.05). Arrow shows time of injection. (J) CTA response in mice exposed to a two-bottle-based test pairing a 5% sucrose solution to rotational stimulus in Vglut2^{VN}:hM4Di mice injected with CNO (n=4) or vehicle (n=3) (t-test, P<0.001).

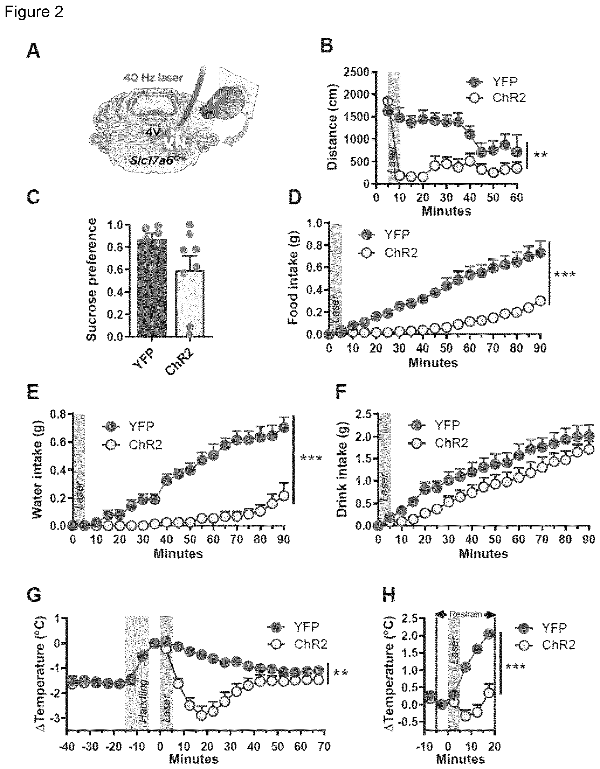

**Figure 2****. VGLUT2^{VN} neuron activation is sufficient to induce kinetosis.** (A) *Slc17a6^{Cre}* mice were unilaterally injected in the right VN with AAV1-DIO-ChR2.YFP (Vglut2^{VN}:ChR2 mice) or AAV1-DIO-YFP (Vglut2^{VN}:YFP mice), followed by optical fiber implantation over the right VN to deliver 40-Hz, 10-ms, 10-mW, 473-nm light pulses for 5 min under different behavioral approaches. (B) Traveled distance during 60 min of open-field test after photostimulation (Vglut2^{VN}:ChR2 mice n=7; Vglut2^{VN}:YFP mice n=5; two-way ANOVA, P<0.01). (C) Conditioned taste aversion (CTA) response in mice exposed to a two-bottle-based test pairing a 5% sucrose solution to optogenetic activation of VGLUT2^{VN} neurons in Vglut2^{VN}:ChR2·YFP (n=8) or control Vglut2^{VN}:YFP (n=6) mice (t-test, P>0.05). (D) Normal-chow intake following laser onset in Vglut2^{VN}:ChR2 or Vglut2^{VN}:YFP mice (n=6; Two-way ANOVA, P<0.001). Animals were food-deprived for 24 h prior to photostimulation. (E) Water intake following laser onset in food-deprived, Vglut2^{VN}:ChR2 or Vglut2^{VN}:YFP mice (n=6; Two-way ANOVA, P<0.001). (F) Highly palatable, chocolate-flavored drink intake following laser onset in food-deprived Vglut2^{VN}:ChR2 or Vglut2^{VN}:YFP animals (n=6; Two-way ANOVA, P>0.05). (G) Core body temperature difference (ΔT) in freely moving Vglut2^{VN}:ChR2 or Vglut2^{VN}:YFP mice after handling and laser stimulation (n=8; Two-way ANOVA, P<0.01). (H) Core body temperature difference (ΔT) after photostimulation in restrained Vglut2^{VN}:ChR2 or Vglut2^{VN}:YFP mice (n=5; Two-way ANOVA, P<0.001). YFP: Vglut2^{VN}:YFP mice; ChR2: Vglut2^{VN}:ChR2 mice.

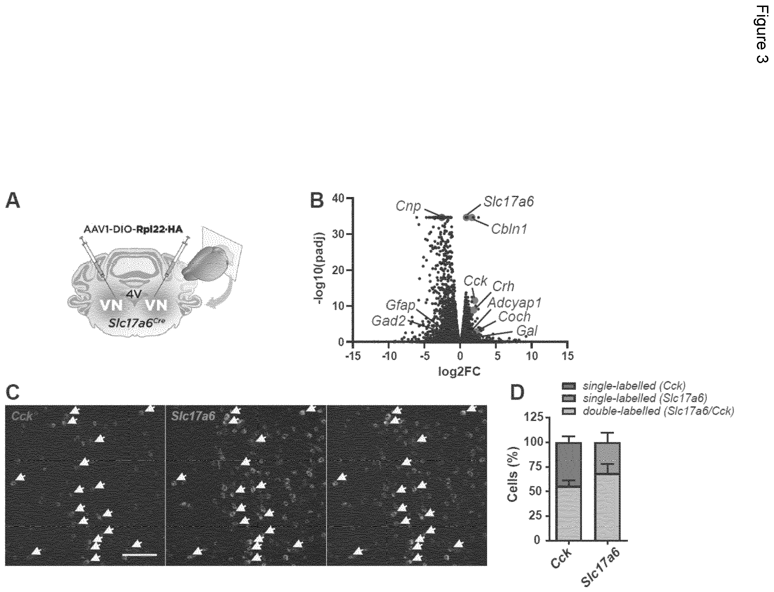

**Figure 3****. Identification of glutamatergic neuronal subpopulations in the VN.** (A) *Slc17a6^{Cre}* mice were bilaterally injected in the VN with a viral vector expressing the RiboTag (AAV1-DIO-Rpl22HA) for the molecular profiling of VGLUT2^{VN} neurons. (B) Differential expression analysis showing significant enrichment for candidate VGLUT2^{VN} neuron subpopulation marker transcripts in the immunoprecipitates of RiboTag assays when compared to inputs (n=3). Specific enrichment for *Slc17a6* (Vglut2), and depletion for inhibitory neuron (Gad2) and non-neuronal marker transcripts (*Cnp, Gfap*) were confirmed in the analysis (padj: adjusted *P*-value; FC: fold change). (C) Double-label *in situ* hybridization assay showing expression of *Cck* mRNA within *Slc17a6*-expressing cells. Scale bar: 100 µm. (D) Percentage of double labelled *Cck-* and *Slc17a6*-expressing cells.

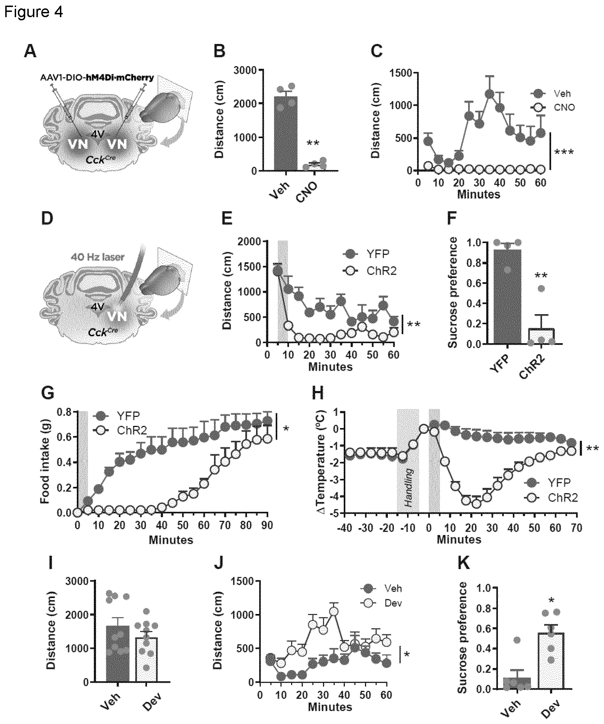

**Figure 4****. CCK^{VN} neuron manipulation affects motion sickness responses.** (A) *Cck^{Cre}* mice were bilaterally injected in the VN with AAV1-DIO-hM4Di.mCherry (Cck^{VN}:hM4Di mice) to inhibit CCK^{VN} neurons upon CNO administration. (B) 5-min open-field test in Cck^{VN}:hM4Di mice 30 minutes after CNO or vehicle administration (n=4; t-test, *P*<0.01). (C) Open-field test in Cck^{VN}:hM4Di mice injected with CNO or vehicle after spin stimulation (n=4; Two-way ANOVA, *P*<0.001). (D) *Cck^{Cre}* mice were unilaterally injected in the right VN with AAV1-DIO-ChR2.YFP (Cck^{VN}:ChR2 mice) or AAV1-DIO-YFP (Cck^{VN}:YFP mice) followed by an optical fiber implantation to deliver a 5-min, 473-nm laser stimulation (40-Hz, 10-mW, 10-ms pulses). (E) Open-field test showing traveled distance after photostimulation in Cck^{VN}:ChR2 and Cck^{VN}:YFP mice (n=4; Two-way ANOVA, *P*<0.01). (F) Conditioned taste aversion (CTA) response in mice exposed to a two-bottle-based test pairing a 5% sucrose solution to optogenetic activation of CCK^{VN} neurons in Cck^{VN}:ChR2·YFP or control (Cck^{VN}:YFP) mice (n=4; t-test, *P*<0.01). (G) Normal-chow intake after light stimulation in Cck^{VN}:ChR2 and Cck^{VN}:YFP mice. Mice were food deprived for 24 h prior to stimulation (n=4; Two-way ANOVA, *P*<0.05). (H) Core body temperature difference (ΔT) in Cck^{VN}:ChR2 and Cck^{VN}:YFP mice after handling and laser stimulation (n=4; Two-way ANOVA, P<0.01). (I) Spontaneous ambulatory activity of mice 45 min after devazepide or vehicle administration in a 5-min, open-field test (n=10; t-test, P>0.05). (J) Traveled distance during 60 min of open-field test after spin stimulation in devazepide or vehicle-treated mice (n=10; Two-way ANOVA, *P* <0.05). (K) Conditioned taste aversion (CTA) response in devazepide or vehicle-treated mice exposed to a two-bottle-based test pairing a 5% sucrose solution to rotational stimulus (n=6; t-test, P-value<0.05).

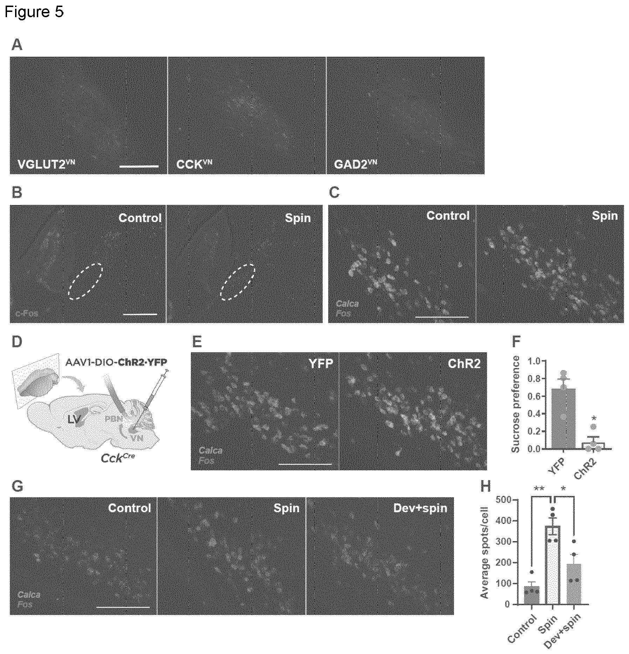

**Figure 5****. CCK^{VN} neurons target the PBN to mediate CTA.** (A) Representative images at Bregma -5.2 mm showing projections to the PBN from VGLUT2^{VN}, CCK^{VN} and GAD2^{VN} neurons as assessed by hM4Di.mCherry visualization in sections containing the PBN from Vglut2^{VN}:hM4Di, Cck^{VN}:hM4Di and Gad2^{VN}:hM4Di mice. Scale bar: 150 µm. (B) c-Fos staining at Bregma -5.2 mm from mice subjected to spin or control stimulation. Dotted line delineates lateral PBN. Scale bar: 400 µm. (C) Double-label *in situ* hybridization assay showing expression of Fos mRNA within Calca-expressing neurons in the PBN after rotational stimulation. Scale bar: 250 µm. (D) Illustration showing AAV1-DIO-ChR2.YFP or AAV1-DIO-YFP injection in the VN and optical fiber implantation over the PBN in *Cck^{Cre}* mice for photoactivation of CCK^{VN} neuron projections in the PBN (CCK^{VN→PBN}). (E) Double-label *in situ* hybridization assay (RNAscope) showing expression of Fos mRNA within Calca-expressing neurons in the PBN after photoactivation of CCK^{VN→PBN} projections. Scale bar: 250 µm. (F) CTA response in mice exposed to a two-bottle-based test pairing a 5% sucrose solution to targeted optogenetic activation of the Cck^{VN→PBN} circuit in Cck^{VN}:ChR2·YFP (n=3) or control (Cck^{VN}:YFP; n=4) mice (t-test, P<0.05). (G) Double-label *in situ* hybridization assay (RNAscope) showing expression of Fos mRNA within Calca-expressing neurons in the PBN of control or spin-stimulated mice with or without devazepide (Dev; 1 mg/kg) administration. Scale bar: 250 µm. (H) Quantification of the average Fos mRNA spots per cell in Calca-expressing neurons in the PBN of control or spin-stimulated mice with or without devazepide (Dev; 1 mg/kg) administration (n=4).

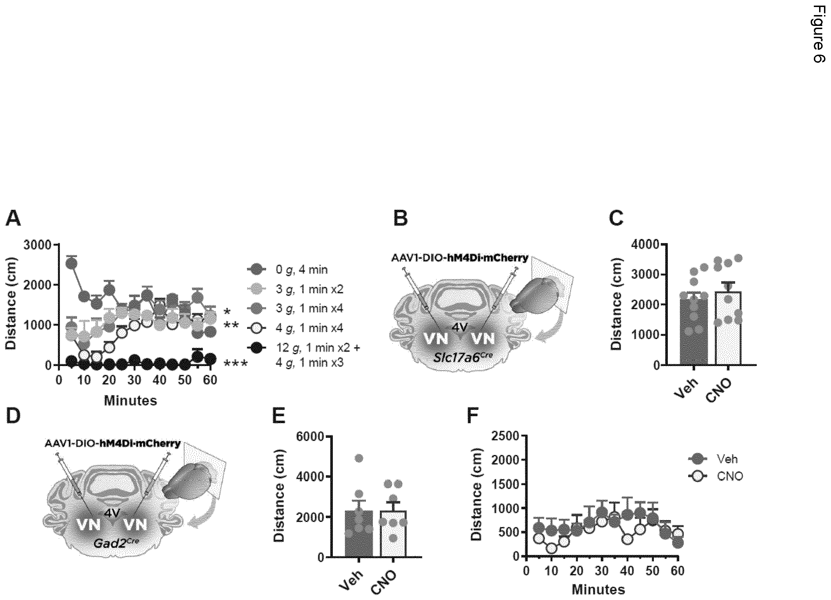

**Figure 6****. Effect of increasing rotational intensities and VGLUT2^{VN} and GAD2^{VN} neuron chemogenetic inhibition on ambulatory activity in mice.** (A) Open-field (OF) test showing reduced locomotor activity in mice subjected to rotational stimuli of different intensity and duration. Centrifugation at 234, 270 and 468 rpm produce a centrifugal force of 3, 4 (n=6) and 12 *g* (n=2), respectively (Two-way ANOVA, *P*<0.001). (B) *Slc17a6^{Cre}* mice were bilaterally injected in the vestibular nuclei (VN) with AAV1-DIO-hM4Di.mCherry (Vglut2^{VN}:hM4Di mice) to inhibit glutamatergic neurons upon CNO administration. (C) Spontaneous ambulatory activity of Vglut2^{VN}:hM4Di mice 30 min after CNO or vehicle administration in a 5-min, open-field test (n=10; t-test, *P*>0.05). (D) *Gad2^{Cre}* mice were bilaterally injected in the vestibular nuclei (VN) with AAV1-DIO-hM4Di.mCherry (Gad2^{VN}:hM4Di mice) to inhibit GABAergic neurons upon CNO administration. (E) Spontaneous ambulatory activity of Gad2^{VN}:hM4Di mice 30 min after CNO or vehicle administration in a 5-min, open-field test (n=7; t-test, P>0.05). (F) Total distance traveled during a 60-min open-field session after spin stimulation in CNO- or vehicle-injected Gad2^{VN}:hM4Di mice (n=7; Two-way ANOVA, *P*>0.05).

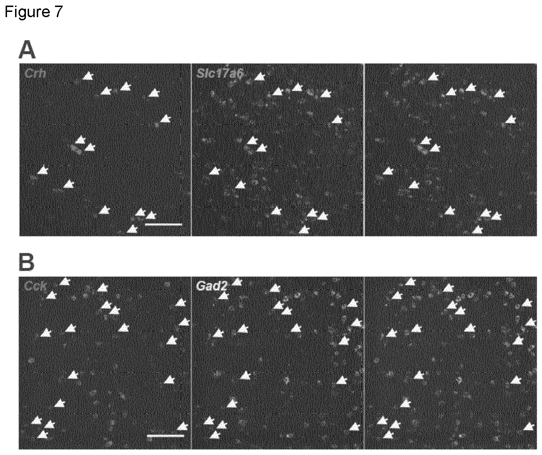

**Figure 7****. Excitatory or inhibitory neuron transcript co-expression in CRH^{VN} or CCK^{VN} neurons.** (A) Double-label *in situ* hybridization assay showing expression of *SIe17a6* mRNA within Crh-expressing cells. Scale bar: 100 µm. (B) Double-label *in situ* hybridization assay showing expression of *Gad2* mRNA within Cck-expressing cells. Scale bar: 100 µm.

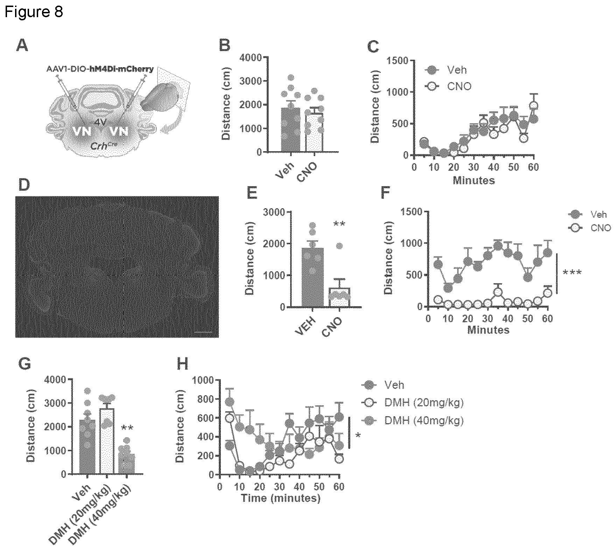

**Figure 8****. Effect of chemogenetic inhibition of CRH^{VN} neurons, CCK^{VN} neurons, and dimenhydrinate administration on ambulatory activity before and after spin stimulation.** (A) *Crh^{Cre}* mice were bilaterally injected in the VN with AAV1-DIO- hM4Di-mCherry (Crh^{VN}:hM4Di mice) to inhibit CRH^{VN} neurons upon CNO administration. (B) Locomotor activity in Crh^{VN}:hM4Di mice (in a 5-min, open-field session) 30 min after CNO or vehicle administration; (n=9; t-test, *P*>0.05). (C) Locomotor activity in Crh^{VN}:hM4Di mice after spin stimulation in CNO and vehicle-injected mice (n=9; Two-way ANOVA, P>0.05). (D) Representative image at Bregma -6.0 mm showing restricted hM4Di mCherry expression in the VN of Cck^{VN}:hM4Di mice injected with reduced amounts (0.2 µl) of the viral vector preparation. Scale bar: 800 µm. (E) 5-min open-field test in Cck^{VN}:hM4Di mice injected with reduced amounts (0.2 µl) of the viral vector preparation, 30 minutes after CNO or vehicle administration (n=6 ; t-test, P<0.01). (F) Open-field test in Cck^{VN}:hM4Di mice injected with reduced amounts (0.2 µl) of the viral vector preparation and CNO or vehicle, after spin stimulation (n=6; Two-way ANOVA, P<0.001). (G) Spontaneous ambulatory activity of mice 30 min after dimenhydrinate (DMH; 20 mg/kg or 40 mg/kg) or vehicle administration in a 5-min, open-field test (n=8; t-test, P>0.05). (H) Traveled distance during 60 min of open-field test after spin stimulation in dimenhydrinate (DMH; 20 mg/kg or 40 mg/kg) or vehicle-treated mice (n=8; Two-way ANOVA, P <0.05).

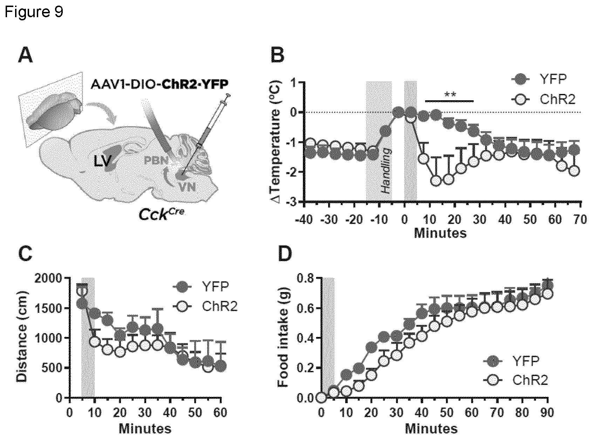

**Figure 9****. Effect of the photoactivation of the CCK^{VN→PBN} projection.** (A) Illustration showing AAV1-DIO-ChR2.YFP or AAV1-DIO-YFP injection in the VN and optical fiber implantation over the PBN in *Cck^{Cre}* mice for photoactivation of CCK^{VN} neuron projections in the PBN (CCK^{VN→PBN}). (B) Core body temperature difference (ΔT) after handling and CCK^{VN→PBN} optogenetic stimulation (Cck^{VN→PBN}:ChR2 mice n=3; Cck^{VN→PBN}:YFP mice n=4; Two-way ANOVA, P<0.01). (C) Open-field test showing traveled distance after CCK^{VN→PBN} photostimulation (n=4; Two-way ANOVA, P>0.05). (D) Food intake in food-deprived mice after CCK^{VN→PBN} photostimulation (n=3; two-way ANOVA, P>0.05).

### Detailed description of the invention

The present invention relates to a cholecystokinin (CCK) antagonist for use in the prevention and/or treatment of a vestibular disease or disorder selected from motion sickness, Meniere's disease, vestibular migraine, disembarkment sickness, labyrinthitis, vestibular neuronitis, benign paroxysmal positional vertigo, labyrinthine infarction and nausea.

Motion sickness, Meniere's disease, vestibular migraine, disembarkment sickness, labyrinthitis, vestibular neuronitis, benign paroxysmal positional vertigo, labyrinthine infarction and nausea share their association with disturbances in the vestibular system, which is responsible for maintaining balance and spatial orientation. These conditions can all involve dysfunction or overstimulation of the inner ear's vestibular apparatus, leading to symptoms such as dizziness, spinning sensations (vertigo), and accompanying nausea. The shared involvement of the vestibular system highlights the interconnectedness of these conditions and their impact on the body's sense of balance and equilibrium.

In a preferred embodiment, said CCK antagonist is selected from a benzodiazepine, benzodiazepinone, and a combination thereof.

In another preferred embodiment, said CCK antagonist is selected from dexloxiglumide, devazepide, TS-941 ((S)-(-)-N-[2,3-dihydro-2-oxo-5-phenyl-1-[(1H-tetrazol-5-yl)methyl] -1H-1,4-benzodiazepine-3-yl]-2-indolecarboxamide), CCK-OPE (_{D}Tyr-Gly-Asp-Tyr(SO₃H)-Nle-Gly-Trp-Nle-Asp-O-phenylethyl ester), JMV179 ((6S,9S,15R,18S,21S)-15-((1H-indol-3-yl)methyl)-9,18-dibutyl-2,2-dimethyl-4,7,10,13,16,19-hexaoxo-21-(phenethoxycarbonyl)-6-(4-(sulfooxy)benzyl)-3-oxa-5,8,11,14,17,20-hexaazatricosan-23-oic acid), Lintitript (2-[2-[[4-(2-chlorophenyl)-1,3-thiazol-2-yl]carbamoyl]indol-1-yl]acetic acid), IQM-95,333 (2-benzyl-5-(N-((tert-butoxycarbonyl)-L-tryptophyl)amino)-1,3-dioxoperhydropyrido(1,2-c)pyrimidine), KSG-504 (4(R)-[N-(3-Methoxypropyl)-N-Pentylcarbamoyl]-5-(2- Naphthylsulfonyl)Pentanoic Acid L-Arginine Salt Monohydrate), T-0632 ([sodium (S)-3-[1-(2-fluorophenyl)-2,3-dihydro-3-[(3-isoquinolinyl)-carbonyl] amino-6-methoxy-2-oxo-1-H-indole]propanoate]) and TP680 ((R)-1-[3-(3-carboxypyridine-2-yl) thio-2-(indol-2-yl)carbonylamino]propionyl-4-diphenylmethyl- piperazine), and a combination thereof.

In an even more preferred embodiment, said CCK antagonist is devazepide.

Regarding the vestibular disease or disorder to be prevented and/or treated according to the present invention, said vestibular disease or disorder is preferably motion sickness.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

As used herein, the term "treat" and its cognates refer to an amelioration of a disease or disorder, or at least one discernible symptom thereof. In another embodiment, "treat" refers to an amelioration of at least one measurable physical parameter, not necessarily discernible by the patient, in another embodiment, "treat" refers to inhibiting the progression of a disease or disorder, either physically (e.g., stabilization of a discernible symptom), physiologically (e.g., stabilization of a physical parameter), or both, in another embodiment, "treat" refers to slowing the progression or reversing the progression of a disease or disorder. As used herein, "prevent" and its cognates refer to delaying the onset or reducing the risk of acquiring a given disease or disorder. In the context of the present invention said "disease or disorder" would be the detrimental events caused by aging of the ovarian reserve of a female mammal.

As used herein, the term "preventing and/or treating" includes "preventing and treating" and "preventing or treating".

The terms "therapeutically effective dose" and "therapeutically effective amount" are used to refer to an amount of a compound that results in prevention, delay of onset of symptoms, or amelioration of symptoms of a disease or disorder. A therapeutically effective amount, as well as a therapeutically effective frequency of administration, can be determined by methods known in the art and discussed below.

In another preferred embodiment, said CCK antagonist according to any of the embodiments herein is combined with at least one another active ingredient.

In a more preferred embodiment, said another active ingredient is a compound selected from an anti-histamine, anti-cholinergic, anti-dopaminergic drug, sympathomimetic and benzodiazepine.

More preferably, said anti-histamine compound is selected from dimenhydrinate, meclizine and promethazine.

More preferably, said anti-cholinergic compound is scopolamine.

More preferably, said anti-dopaminergic drug is selected from prochlorperazine and metoclopramide.

In another preferred embodiment, the CCK antagonist according to any of the embodiments herein is included in a pharmaceutical composition which may also include at least one another active ingredient. Said composition may further comprise a pharmaceutically acceptable vehicle, adjuvant, diluent or excipient, such as preserving agents, fillers, disintegrating agents, wetting agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated.

In another preferred embodiment, the CCK antagonist according to any of the embodiments herein is included, alone or combined with at least one another active ingredient, in a functional food or a dietary supplement. "Alone" is meant to be as the unique active ingredient but including any acceptable vehicle, adjuvant, diluent or excipient suitable for such functional food or dietary supplement.

In another preferred embodiment of the present invention, said prevention and/or treatment comprises orally, intravenously or intraperitoneally, preferably orally, administering the CCK antagonist according to any of the embodiments herein or the pharmaceutical composition comprising thereof.

The present disclosure also relates to a method of preventing and/or treating a vestibular disease or disorder selected from motion sickness, Meniere's disease, vestibular migraine, disembarkment sickness, labyrinthitis, vestibular neuronitis, benign paroxysmal positional vertigo, labyrinthine infarction and nausea, preferably motion sickness, according to the embodiments included herein, comprising the step of administering a therapeutically effective amount of a cholecystokinin (CCK) antagonist, preferably selected from a benzodiazepine, benzodiazepinone, and a combination thereof. In more preferred embodiment said CCK antagonist is selected from dexloxiglumide, devazepide, TS-941, CCK-OPE, JMV179, Lintitript, IQM-95,333, KSG-504, T-0632 and TP680 and a combination thereof, and in an even more preferred embodiment said CCK antagonist is devazepide.

The compound of the present invention is capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition. The compound as provided herein can be formulated into pharmaceutical compositions by admixture with one or more pharmaceutically acceptable vehicle, adjuvant, diluent or excipient. Such compositions may be prepared for use in oral administration, particularly in the form of tablets or capsules; or parenteral administration, particularly in the form of liquid solutions, suspensions or emulsions.

In the case of oral administration, the tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. Capsules can be in the form of a hard capsule or soft capsule, which are generally made from gelatine blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bimodal.

Liquid preparations for administration include sterile aqueous or non-aqueous (for example, ethanol or DMSO) solutions, suspensions, and emulsions. The liquid compositions may also include binders, buffers, preservatives, chelating agents, sweetening, flavouring and colouring agents, and the like.

A number of examples will be provided below that are intended to illustrate the invention and in no way limit the scope of the invention, which is established by the attached claims.

### EXAMPLES

### METHODS

### Mice

The following mouse lines were used in this study: *Slc17a6^{Cre}* (BAC-Vglut2-Cre) (12) mice were generated by Ole Kiehn. *Cck^{Cre}* (CCK-IRES-Cre) and *Crh^{Cre}* (CRH-IRES-Cre) (62) mice were obtained from The Jackson Laboratory (Bar Harbor, ME. Stock No: 012706 and 012704, respectively). Mice were group-housed with a 12:12h light:dark cycle at 22°C, with *ad libitum* access to rodent chow (Teklad Global Rodent Diet #2014S; Envigo) and water, unless otherwise stated. Sex and age-balanced groups of 2- to 7-month-old mice were used across all experimental procedures. No sex differences were observed. After surgeries, animals were individually housed until the end of all experimental procedures. Sample sizes were determined using power analyses. Number of animals used per experiment (n) are detailed in figure legends. All mice were on a C57BU6J background after backcrossing for at least 10 generations. All experiments were conducted following the recommendations in the Guide for the Care and Use of Laboratory Animals and were approved by the Animal Care and Use Committee of the Universitat Autònoma de Barcelona and the Generalitat de Catalunya.

### Drugs

Devazepide (Dev; 1 mg/kg in saline solution containing 1% DMSO and 1% Tween-80) and dimenhydrinate (DMH; 20 mg/kg or 40 mg/kg in saline solution) were administered intraperitoneally (i.p.) 45 or 30 minutes before tests, respectively.

### Rotational stimulus

Prior to rotational stimulation, animals were habituated to physical restraint for 4 min using a 50-mL conical tube coupled to a custom-made rotary device (external radius: 10.5 cm; internal radius from mouse head: 5 cm. Rotation multiplier: x3.6). Afterwards, rotational (four repeated 1-min, 4-g accelerations) or control (4 min with no rotation) stimuli were applied, unless otherwise stated. 1-min accelerations included 55 s of rotation plus 5 s break until full stop. To achieve 4-g accelerations, 75 rpm were applied.

### Behavioral assays

Each animal was subjected to an open-field test, followed by food intake and conditioned taste aversion (CTA) analysis. After tests, telemetric temperature sensors were implanted, and core-body temperature monitored.

### Open-field test

The open-field (OF) test was conducted in a non-covered, white methacrylate box (56 x 36.5 x 31 cm) that allows for video recording during animal testing. Mice were individually exposed for 5 min to the OF before receiving rotational or control stimulation. After stimulation, mice were re-exposed to the OF for 60 more min. For optogenetic experiments, mice were exposed for a total of 60 min to the OF (5 min of pre-stimulation, followed by 5 min of laser stimulation and 50 min of post-stimulation). Spontaneous ambulatory activity was monitored using a video tracking software (Ethovision XT 11.5; Noldus Information Technology).

### Food and liquid intake measurement

Animals were individually placed in an Oxyletpro-Physiocage monitoring system (Panlab) for real-time quantification of food and liquid intake. Mice were habituated to the cage for 2 to 4 days with *ad libitum* access to normal chow and water, unless otherwise stated. Prior to each session, animals were food deprived for 24 h and refed at the onset of the dark cycle. For specific experiments, chocolate-flavored, highly palatable liquid diet (ENSURE Nutrivigor, Abbott) was provided during cage habituation and in the subsequent experimental sessions. Food and liquid intake were expressed as cumulative intake using the Metabolism software version v3.0.00 (Panlab).

### Conditioned taste aversion (CTA) test

For the CTA test, a two bottle-based protocol was used (17). Animals were individually placed in a custom cage with angular ports for two liquid-containing bottles. *Ad libitum* water access was provided for 2 days during habituation. Next, animals only had access to both water-containing bottles during restricted time periods (30 min access in the morning, 30-60 min in the afternoon) for 8 days (D1- D8). During days 4 and 6 (D4 and D6), a solution of 5% sucrose in water was paired with specific stimuli (rotation, CNO injection, photostimulation) to stablish conditioning. Mice were tested on D8. Sucrose preference value was calculated as sucrose solution consumption/total liquid consumption.

### Surgical implantation of telemetry devices and temperature monitoring

Anesthetized mice (5% isoflurane for induction, 1.5% for maintenance) were aseptically implanted with telemetric temperature transmitters (G2 E-Mitter, STARR Life Sciences Corp.) into the peritoneum and allowed to recover for 2 weeks. Mouse cages were placed on telemetry receivers (ER4000 Energizer/Receiver, STARR Life Sciences Corp.) and core body temperature was monitored using the VitalView software version 5.0 (STARR Life Sciences Corp.) under controlled ambient temperature (22°C). All recordings started in resting animals. For specific experiments, mice were inserted into a modified 50-mL Falcon tube to ensure physical restrain while allowing optogenetic tethering.

### Viral vector production

pAAV-hSyn-DIO-hM4Di:mCherry, pAAV-EF1a-DIO-eYFP and pAAV-EF1a-DIO-ChR2:YFP plasmids were obtained from Addgene (#44362, #27056 and #100056, respectively). pAAV RiboTag virus (pAAV-EF1a-DIO-Rpl22·HA) has been described (Sanz et al., 2015). Recombinant adeno-associated viral vectors (AAV) were produced in human embryonic kidney (HEK293T) cells with AAV1 coat serotype. Purification was achieved by several sucrose and CsCI gradient centrifugations and a final resuspension in 1x Hanks Balanced Saline Solution (HBSS) at a titer of 2 x 10⁹ viral genomes/µL as described (51, 56). AAV preparations were aliquoted and stored at - 80°C until stereotaxic injection.

### Stereotaxic surgery

All surgeries were performed under aseptic conditions. Animal anesthesia was induced and maintained with 5% and 1-1.5% isoflurane/O₂, respectively. Analgesia (5 mg/kg ketoprofen; Sanofi-Aventis) and ocular protective gel (Viscotears^{®}, Bausch+Lomb) were applied. Mice were then placed over a heating pad in a robot-operated, 3-dimensional (stereotaxic) frame (Neurostar) for intracerebral virus delivery. Stereotaxic coordinates were normalized using a correction factor (Bregma-Lambda distance/4.21) based on the coordinates of Paxinos and Franklin (20). AAV preparations were unilaterally (right side) or bilaterally delivered into the VN (antero-posterior (AP), -6.00 mm; medio-lateral (ML), ± 0.90 mm; dorso-ventral (DV), -4.00 mm from Bregma) at a constant rate of 0.1 µL/min for 3.5-4.0 min (0.20-0.40 µL per injection site) using a 32-gauge blunt needle coupled to a 5 µL-syringe (Hamilton). After infusion, the needle was maintained in place for 6 min to allow proper diffusion. Subsequent needle withdrawal was performed at 1 mm/min to ensure minimal off-target viral leakage. After viral injection, mice used for optogenetic experiments also received unilateral surgical implantation of a fiber-optic cannula as described below.

### Chemogenetics

*Sle17a6^{Cre}, Cck^{Cre}* or *Crh^{Cre}* mice were bilaterally injected in the VN with 0.35 µL (per side) of AAV1-hSyn-DIO-hM4Di:mCherry. Clozapine-n-oxide (CNO; 1 mg/kg) was administered via intraperitoneal (i.p.) injection 35 min prior to rotational or control stimulation.

### Optogenetics

*Slc17a6^{Cre}* or *Cck^{Cre}* mice were injected in the right VN with 0.4 µL of either AAV1-EF1α-DIO-ChR2·YFP or AAV1-EF1α-DIO-eYFP and a fiber-optic cannula (200-µm fiber core diameter, 0.22-numeric aperture; 2.5-mm ferrule diameter; Thorlabs) was implanted over the right VN (AP, -6.00 mm; ML, +0.90 mm; DV, -4.00 mm) or over the right PBN (AP, -5.20 mm; ML, +1.70 mm; DV, -3.00 mm) of *Cck^{Cre}* animals. A fiber-optic cannula was fixed to the exposed skull with a layer of adhesive cement (Super-Bond C&B, Sun Medical) and dental acrylic cement (Rebaron, GC Corporation). The skin was affixed to the cement with tissue adhesive (Vetbond, 3M). A blue 473-nm laser light was produced by a DPSS Laser System (LRS-0473-GFM-00100-05 Laserglow) and driven by a fiber-optic patch cord (200-µm core diameter, 0.22-numeric aperture: FT030 protection, Thorlabs). Light intensity was set at 10 mW measured by a photometer (Thorlabs) at the tip of a non-implanted fiber optic cannula attached to the patch-cord. To deliver illumination to the right VN or right PBN, the patch cord was connected to the implanted fiber-optic-containing cannula through a ceramic sleeve. A pulse generator (33500B Series Trueform, Keysight) was used to adjust laser output to deliver 40-Hz, 10-ms, pulse trains for 5 min to all mice.

### Mapping of neuronal projections

To visualize the projections of VGLUT2^{VN}, CCK^{VN} or GAD2^{VN} neurons, *Sle17a6^{Cre}*, *Cck^{Cre}* or *Gad2^{Cre}* mice were injected into the right VN with 0.2 µL of AAV1-hSyn-DIO-hM4Di:mCherry. Animals were euthanized 3 weeks after surgery for direct visualization of fibers after tissue fixation and cryopreservation.

### Tissue processing and Immunofluorescence analysis

Mouse brains were freshly dissected following euthanasia by CO₂ asphyxia, fixed overnight with phosphate-buffered saline (PBS) containing 4% paraformaldehyde (PFA) and cryo-protected with 30% sucrose in PBS. For cryo-sectioning, brains were frozen for 5 min in dry ice and sectioned in a freezing microtome. For immunofluorescence, 30-µm free-floating sections were blocked in PBS with 10% normal donkey serum (NDS) and 0.2% Triton X-100 for 1 h at room temperature followed by an overnight incubation at 4°C with primary antibody solution containing a rabbit anti-c-Fos (1:1,000; #ab222699, Abcam) antibody in PBS with 1% NDS and 0.2% Triton X-100. After three washes in PBS with 0.2% Triton X-100, a secondary antibody solution containing a secondary antibody conjugated to Alexa Fluor 594 fluorophore (1:500; Invitrogen) was added to the sections and incubated for 1 h at room temperature. After the incubation, sections were washed three times for 5 min in PBS with 0.2% Triton X-100 and mounted onto slides with DAPI Fluoromount (#17984-24, Electron Microscopy Sciences) before visualization with an EVOS imaging system (Thermo Fisher Sci).

### RiboTag assays

For genetic identification of neuronal subsets, *Slc17a6^{Cre}* mice were bilaterally injected in the VN with 0.4 µL of the RiboTag viral vector (AAV1-DIO-Rpl22HA) (56). Animals were euthanized 3 weeks after surgery for subsequent RiboTag analysis. To isolate the polysome-associated mRNAs from VGLUT2^{VN} neurons, punches containing the VN of *Slc17a6^{Cre}* mice injected with AAV1-DIO-Rpl22·HA were pooled and homogenized in 1 ml of buffer as described (57). After centrifugation, 4 µL of anti-HA antibody (MMS-101R, 2-3 mg/ml; Covance) was added to 800 µL of the cleared lysate and incubated for 4 h at 4°C. Remaining lysate was saved as input sample. After incubation, 200 µL of protein A/G magnetic beads (Thermo Scientific) were added and incubated overnight at 4°C with rotation. Immunoprecipitates (IPs) were washed in high-salt buffer and RNA from inputs and IPs were extracted (55). For differential expression analysis, 10 ng of RNA was amplified using the Ovation Pico SL WTA system (NuGEN). Fidelity of amplification was confirmed by qPCR analysis of the resulting cDNA using the QuantiTect kit (Qiagen) before biotinylation according to the EncorelL biotinylation kit (NuGEN). Biotinylated cDNA was quantified, and product size distribution was analyzed using the 2100 Bioanalyzer system with the RNA 6000 Nano chips (Agilent Technologies). Biotinylated cDNA (750 ng) was hybridized at 48°C to MouseRef-8 v2 expression beadchips (Illumina) for 16 h before washing and analyzing according to the manufacturer's directions. Signal was detected using a BeadArray Reader (Illumina), and data were analyzed for differential expression using the GenomeStudio data analysis software (Illumina). Average normalization, the Illumina custom error model, and multiple testing corrections using the Benjamini-Hochberg false discovery rate were applied to the analysis. Only transcripts with a differential score of >13 (p < 0.05) were considered. Normalized and raw data have been deposited in the National Center for Biotechnology Information Gene Expression Omnibus and are accessible through GEO Series accession number GSE167672 .

### In situ hybridization assays

Mouse brains were fresh-frozen in Tissue-Tek O.C.T. compound (Sakura) with dry ice and stored at -80 °C until cryosectioning. Coronal sections (15 µm) containing the VN or the PBN were used for RNAscope (Advanced Cell Diagnostics) analysis following manufacturer's directions. The following probes were used: Mm-SIc17a6 (#319171-C3), Mm-Cck (#402271-C2), Mm-*Crh* (#316091-C1), Mm-Gad2 (#439371-C1), Mm-Fos (#316921-C1) and Mm-Calca (#578771-C2). All *in situ* hybridization assays were imaged using a confocal (Leica SP5) or epifluorescence (Nikon Eclipse 90i) microscope and analyzed in imaged (Fiji v1.0) or QuPath open-source software (Bankhead et al., 2017). Cell counting was performed in VN sections from Bregma - 5.68mm to -6.64mm in 2-3 slices/animal (n=3 animals) with imaged Cell Counter plugin (Fiji v1.52). Number of Fos probe spots *per* Calca-expressing cell was analyzed using QuPath. Cell segmentation was accomplished in DAPI-stained sections containing Calca-expressing neurons from Bregma -5.02 mm to -5.34 mm using the cell detection feature with a cell expansion of 30 µm. After cell detection, Fos and Calca transcripts were detected using the subcellular detection module. Each section was visually inspected to confirm accurate spot identification. Number of Fos probe spots per Calca-expressing cell was obtained from 2-5 sections per mice. 4 animals were used per group.

### Statistics

Data are shown as the mean ± SEM. GraphPad Prism v9.0 software was used for statistical analyses. Appropriate tests were selected depending on the experimental design as stated in the figure legends. Statistical significance, when reached (p<0.05 was considered significant), is stated in the figure legends.

### RESULTS

### Glutamatergic vestibular neurons sustain motion-induced autonomic responses

Assessment of MS in mice is hindered by the lack of emetic reflex and difficulty to unequivocally identify nausea (42, 49, 59) . However, behavioral and autonomic signs of MS are consistently observed in mice subjected to rotatory or gravitational paradigms 1, 38, 42, 69). Thus, we sought to establish a rotational paradigm sufficient to robustly develop MS-like signs (Figure 1A). Four repeated 1-min, 4-g accelerations (spin stimulation) consistently induced transient hypolocomotion (Figure 1B and 6A). Furthermore, a significant decrease in food intake was observed in food-deprived mice after spin stimulation compared to controls (Figure 1C), in line with the appetite suppression observed in MS (1, 21, 36, 39, 42, 69). In addition, a decrease in core body temperature, another classical MS response (21, 36, 38, 39, 40, 42), was observed after the rotational stimulus compared to controls, with a maximum temperature difference of 4°C that occurred -17.5 min after rotation onset (Figure 1D).

MS is known to induce a robust CTA (13). Hence, we asked whether rotation could establish a CTA in a two-bottle choice test (17). Two conditioning sessions pairing a 5% sucrose solution with rotational motion, were followed by a choice test of water or sucrose two days later. We observed a significant reduction in sucrose preference when sucrose was paired with rotation, compared to controls (Figure 1E).

With the validity of the rotation paradigm established, we set out to define the neuronal substrate of vestibular-induced, MS-like responses. Excitatory neurons are highly abundant in the VN (19) and glutamatergic VN neurons are activated after provocative motion (14). Among the different glutamatergic markers, VGLUT2 (encoded by *SIc17a6)* shows a robust and significant expression in the VN (41), and VGLUT2-expressing neurons have been shown to participate in vestibular responses to hypergravity and postural imbalance, in contrast to GABAergic (VGAT-expressing) inhibitory neurons (2, 37). Thus, we assessed the necessity of genetically defined glutamatergic VGLUT2-expressing VN (VGLUT2^{VN}) neurons in eliciting MS behavioral and autonomic responses by bilateral chemogenetic inhibition in a well-characterized *Slc17a6^{Cre}* mouse line (12) (Figure 1F, Figure 6B). Targeted chemogenetic inhibition of VGLUT2^{VN} neurons by expression of hM4Di and administration of clozapine N-oxide (CNO) did not induce significant effects on locomotion prior to spin stimulation (Figure 6C). However, inhibition of VGLUT2^{VN} neurons by CNO administration prevented spin-induced decreases in ambulatory activity (Figure 1G), appetite suppression (Figure 1H) and attenuated the MS-related decrease in body temperature (Figure 1I). Noteworthy, an initial decrease in body temperature was observed in CNO-injected mice, suggesting that other neuronal populations may contribute to the early spin-induced drop in body temperature. In addition, inhibition of VGLUT2^{VN} neurons also prevented spin-induced CTA (Figure 1J). In contrast, bilateral chemogenetic inhibition of GABAergic (Gad2 -expressing) VN neurons did not alter spin-induced hypolocomotion (Figure 6D-F). These results indicate that VGLUT2^{VN} neurons are necessary to promote the development of the MS-like behavioral and autonomic responses elicited by rotational stimulation.

Nauseogenic responses can be obtained after unilateral inner ear caloric stimulation (32). Hence, to test whether VGLUT2^{VN} neuronal activation is sufficient to induce MS-like autonomic responses, *Slc17a6^{Cre}* mice received a unilateral injection of an AAV1 expressing Cre-dependent ChR2 YFP (Vglut2^{VN}:ChR2 mice; for photoactivation) or a YFP construct (Vglut2^{VN}:YFP mice; as control) into the right VN, and an optical fiber was implanted over the injected VN (Figure 2A). We used a 5-min, 40-Hz, optogenetic-stimulation paradigm because we and others have shown VGLUT2^{VN} neurons in actively moving mice maintain firing rates of up to 40 Hz (11, 64). VGLUT2^{VN} neuron optogenetic activation led to a significant decrease in spontaneous ambulatory activity (Figure 2B), consistent with the results obtained after rotational stimulus. Pairing 5% sucrose solution to unilateral optogenetic activation of VGLUT2^{VN} neurons did not result in significant differences between the ChR2 and the YFP group (Figure 2C), even though ingestion of their regular, low-calorie chow and water intake were significantly decreased after optogenetic VGLUT2^{VN} activation, showing complete suppression for as long as 30 min after laser onset (Figures 2D and 2E). Noteworthy, a normal feeding pattern was observed after presentation of highly palatable, chocolate-flavored, liquid diet to a separate cohort of laser-stimulated Vglut2^{VN}:ChR2 mice (Figure 2F), ruling out physical inability to feed but rather a lack of motivational drive to consume regular chow or water. Core body temperature significantly decreased, with a maximum 3°C-drop occurring 17.5 min after laser onset (Figure 2G). To assess whether the observed optogenetic-induced loss of core body temperature was due to a reduction in ambulatory activity in Vglut2^{VN}:ChR2 mice, an additional optogenetic stimulation was applied under physical restraint. Under these conditions, core body temperature increased in Vglut2^{VN}:YFP mice, likely due to restraint-induced stress responses. However, Vglut2^{VN}:ChR2 mice still showed a significant drop in body temperature coincident with photostimulation (Figure 2H). Thus, these results highlight that VGLUT2^{VN} neuron activation is sufficient to recapitulate most MS-induced symptoms.

### Identification of Crh- and Cck-expressing VGLUT2^{VN} neuronal subpopulations

To define VGLUT2^{VN}-neuron subpopulations, we performed viral vector-mediated RiboTag molecular profiling (55, 56, 57) in VN homogenates of *Slc17a6^{Cre}* mice injected with a RiboTag-expressing construct (AAV1-DIO-Rpl22·HA ((56); Figure 3A). Transcripts enriched in VGLUT2^{VN} neurons were identified by differential expression analysis in RNA samples extracted from the RiboTag immunoprecipitates (IP; containing polysome-associated mRNAs from VGLUT2^{VN} neurons) and the input (I) of the immunoprecipitation (containing RNA from all the different cell types in the VN). Data analysis confirmed specific enrichment for *Slc17a6* (Vglut2), and depletion of inhibitory neuron mRNAs (Gad2) and non-neuronal transcripts (*Cnp*, *Gfap*) in the RiboTag Ips. In addition, there was a significant enrichment for candidate VGLUT2^{VN} neuron subpopulation markers such as *Cck*, *Crh*, *Adcyap1*, *Gal*, *Cbln1* and *Coch* (Figure 3B).

Among these, *Crh-* and Cck-expressing neurons have been shown to be involved in autonomic and nauseogenic responses (43, 47), highlighting the potential relevance of these neuronal populations in VN-mediated MS responses. Subsequent *in situ* hybridization assays confirmed the existence of scattered Crh-positive cells that colocalized with *Slc17a6* (Figure 7A). On the other hand, an abundant population of *Cck-*expressing neurons was detected, constituting over 60% of all *Slc17a6-* expressing neurons (Figure 3C,D). Noteworthy, a second population of Cck-expressing cells (-45% of the total) that did not co-localize with *Slc17a6* was identified (Figure 3C,D) and co-localization with Gad2 was observed (Figure 7B).

### Modulation of vestibular Cck-expressing neurons causes MS-like autonomic responses

To test the necessity of vestibular *Crh*- or Cck-expressing neuronal populations (CRH^{VN} or CCK^{VN} neurons, respectively) in the development of MS-like signs, we injected *Crh^{Cre}* or *Cck^{Cre}* mice bilaterally in the VN with a AAV vector carrying Cre-dependent hM4Di-mCherry (Figure 8A and 4A). Chemogenetic inhibition of CRH^{VN} neurons did not induce any effect pre- or post-spin on ambulatory activity (Figure 8B,C), indicating that they are not necessary to develop rotation-induced autonomic responses. Unexpectedly, bilateral chemogenetic inhibition of CCK^{VN} neurons significantly decreased ambulatory activity in the absence of rotational stimulus (Figure 4B), which led to an inability to move after the spin (Figure 4C). Reducing the volume of the inhibitory chemogenetic vector (from 0.35 µl to 0.2 µl) in the VN of *Cck^{Cre}* mice also led to a marked decrease in locomotion in the CNO-injected hM4Di group, ruling out a role for Cck-expressing neurons from neighboring nuclei (Figure 8D-F). Since CCK^{VN} neuron inhibition *per se* is sufficient to induce locomotor effects, further experiments using this approach were hindered. Thus, we sought to establish if stimulation of CCK^{VN} neurons would be sufficient to elicit MS-like responses. Optogenetic activation of CCK^{VN} neurons (Figure 4D), led to a significant and prolonged decrease in spontaneous ambulatory activity in the open-field test compared to the control animals (Figure 4E) and the appearance of a robust CTA (Figure 4F). Similarly, food intake was significantly decreased after photostimulation of CCK^{VN} neurons, showing a complete suppression for 35 min, while control animals engaged in feeding almost immediately (Figure 4G). Optogenetic stimulation of CCK^{VN} neurons reduced core temperature (~4.5°C), occurring 22.5 min after laser onset, in contrast to control mice (Figure 4H). Thus, our results underscore that stimulation of CCK^{VN} neurons is sufficient to elicit MS-like behavioral and autonomic responses.

Since global CCK^{VN} inactivation alters normal VN function, likely by the combined action of neurotransmitters and neuropeptides, we also assessed the specific role of CCK signaling blockade in spin-induced responses (Figure 4I-K). Systemic administration of devazepide (Dev), a CCK-A receptor antagonist, prior to the rotation stimulus, was sufficient to block hypolocomotion (Figure 4J) and the appearance of CTA (Figure 4K), without having any overt behavioral pre-spin effect (Figure 4I). In comparison, administration of the common anti-motion sickness antihistamine dimenhydrinate (DMH) was able to ameliorate spin-induced hypolocomotion only at 40 mg/kg, a dose that reduced locomotion prior to the rotatory stimulus (Figure 8G-H).

These results reveal that vestibular CCK signaling can mediate MS-like behavioral and autonomic responses.

### A CCK^{VN→PBN} circuit mediates MS-like CTA via CGRP neuron activation

We hypothesized that specific vestibular projections were likely driving discrete MS-induced responses. To elucidate the genetically defined, vestibular outputs involved in these responses, we compared the vestibular projections described in the Allen Mouse Brain Connectivity Atlas (121146455, 300687607) to the projection fields of *Sle17a6^{Cre}*, *Cck^{Cre},* and *Gad2^{Cre}* mice injected with a Cre-dependent hM4Di-mCherry construct. Among the different targeted areas, the PBN was consistently targeted in all animal groups (Figure 5A), in agreement with the Allen Mouse Brain Connectivity Atlas and prior reports (4, 5, 14, 27, 34, 58). These data confirmed the PBN as a terminal field for VN neurons.

Our results pointed at an excitatory role for vestibuloparabrachial inputs. Accordingly, a rotational stimulus induced neuronal activation as assed by c-Fos staining in the PBN (Figure 5B). To identify the neuronal population in the PBN receiving inputs from VN neurons, we performed double-label ISH assays for Fos and Calca (which encodes the calcitonin gene-related peptide, CGRP) because these PBN neurons have been implicated in mediating visceral malaise and CTA (15, 16). Results showed that spin stimulation induced Fos expression in Calca-expressing neurons (Figure 5C). Thus, we hypothesized that a Cck-expressing vestibuloparabrachial (CCK^{VN→PBN}) circuit might be relevant in MS by impinging onto CGRP^{PBN} neurons. To that end, we optogenetically activated CCK^{VN} axon terminals in the ipsilateral PBN by placing the optical fiber-tip over this region in Cck^{VN}:ChR2 mice (Figure 5D and 9A). Photoactivation of CCK^{VN} neuronal terminals increased the number of Fos positive-cells in the PBN, which colocalized in part to Calca-expressing neurons (Figure 5E).

Behaviorally, optogenetic stimulation of CCK^{VN→PBN} fibers at 40Hz significantly decreased sucrose preference when compared to controls (Figure 5F), as well as body temperature (Figure 9B) without affecting locomotion or food intake (Figure 9C,D). Overall, these results underscore CCK^{VN→PBN} activation of CGRP neurons as a key component of MS-induced CTA. Antagonism of CCK-A receptor by devazepide administration led to a significant decrease in the activation of CGRP^{PBN} neurons as assessed by double ISH assays for Fos and Calca transcripts (Figure 5G,H), identifying a role for CCK-A signaling for the development of MS responses.

### DISCUSSION

Our results link VGLUT2^{VN} and CCK^{VN} neuronal populations to the development of MS-like symptoms in mice. Furthermore, we reveal a CCK^{VN→PBN} projection that is sufficient to induce CTA and hypothermia likely through the activation of an ensemble of PBN neurons that express the CCK-A receptor including Calca-expressing neurons. Our results suggest the potential use of CCK-A receptor blockers as a treatment for MS.

MS is conserved among animal phyla (24, 39, 42, 63, 65), but with a high degree of heterogeneity in its behavioral and physiological correlates. Mice do not present an emetic reflex but do present overt physiological and behavioral alterations when exposed to a nauseogenic experience (13, 21, 38, 39, 66, 69) such as sustained 2-g rotational stimuli (1, 2, 21, 38, 42, 69). We show that an intermittent rotational stimulus of 4 min is sufficient to develop robust hypolocomotion, hypophagia, hypothermia, and CTA.

The role of the VN in the development of MS is well recognized (1, 38). The currently accepted hypothesis is that the vestibular inner ear organs provide a major input for the subsequent computing comparisons between present sensory input (integrated input including vestibular, visual and proprioceptive information) and memory recalled from similar motion situations experienced in the past (45, 52). Rotational stimuli convey information from the semicircular canals mostly onto medial VN (MVN) neurons, leading to nauseogenic responses (32, 49, 50). Previous studies had identified that VGLUT2^{VN} neurons, highly abundant in the VN (19) participate in postural balance and mediate gravitational stress -induced hypolocomotion, hypophagia, and hypothermia (2, 37). Here, we confirm these results and show, for the first time, that VGLUT2^{VN} neurons are necessary for the development of MS responses, such as CTA, in a rotational paradigm in mice.

Several excitatory populations have been described in the VN that may be involved in different aspects of vestibular-mediated responses (30). Using the RiboTag approach (55, 57) we identified several genetic markers for subpopulations. Our results validate genes *Crh* and *Adcyap1* (30) and identify new markers for VGLUT2^{VN} neurons including *Gal, Coch* and *Cck*. Furthermore, we provide evidence that Cck-expressing neurons are the most abundant glutamatergic vestibular neuronal population.

At the functional level, we show that unilateral optogenetic activation of CCK^{VN} neurons is sufficient to recapitulate MS-induced responses, such as hypolocomotion, hypothermia, hypophagia and to elicit a robust CTA. On the other hand, restricted chemogenetic inhibition of CCK^{VN} neurons significantly decreased ambulatory activity and temperature, even in the absence of a rotational stimulus.

Different mechanisms may account for this paradoxical response. First, VN presents a high degree of compensation, such as contralateral commissural inhibition (35). Thus, alterations in neuronal activity may lead to compensatory contralateral activation. However, these responses were neither observed after VGLUT2^{VN}, GAD2^{VN} nor CRH^{VN} inhibition, pointing at a specific role of CCK^{VN} neurons. In this regard, we have uncovered the existence of both glutamatergic, and GABAergic CCK^{VN} neurons. This fact, given the existence of GABAergic local and commissural interneurons as well as GABAergic projection populations (Highstein & Holstein, 2006) may underlie the strong effect observed after CCK^{VN} activity modulation. Alternatively, MVN neurons produce endogenous, spontaneous pacemaker activity (33). Restoration of MVN pacemaker activity is key for MS habituation (53). Thus, it may be possible that CCK ^{VN} neurons contribute to pacemaker activity and alterations in their rhythmic firing rate may lead to MS-like symptoms.

At the circuitry level, our results underscore genetically defined, target- and density-specific projections that may underlie their differential contribution to MS-induced responses. Of the different axonal projections, we describe dense projections from CCK^{VN} neurons to the PBN. The PBN is known to mediate malaise, appetite suppression, lethargy, anxiety, thermoregulation, and CTA (44, 46, 66). Chemogenetic inhibition of VGLUT2^{VN} neurons blocked hypothermia and CTA in our MS paradigm. Unilateral optogenetic stimulation of CCK^{VN} neurons or their projections to the PBN produced hypothermia and robust CTA. Activation of VGLUT2^{VN} neurons leads to hypothermia, likely by decreasing sympathetic tone (2). Recent reports revealed that Pdyn-expressing PBN neurons (Pdyn^{PBN}) regulate hypothermia through projections to the pre-optic area (44), involved in temperature regulation (61). Hence, CCK^{VN} activation of Pdyn^{PBN} neurons might mediate the MS-induced drop in body temperature.

Rotational stimulus and CCK^{VN} optogenetic stimulation activated CGRP-expressing, glutamatergic neurons in the lateral PBN, which are known to be involved in malaise, and CTA (Palmiter, 2018), sustaining the establishment of aversive taste memories (17). Thus, we propose that glutamatergic CCK^{VN} input onto CGRP^{PBN} neurons mediates MS-induced CTA. CCK^{VN→PBN} projections do not seem to participate in MS-induced hypophagia and hypoactivity, even though activation of CGRP^{PBN} neurons is involved in appetite suppression and reduced locomotion (16, 46) . Thus, it is likely that CCK^{VN} projections to other brain areas may be responsible for these responses. For example, CCK^{VN} neurons also project to the NTS, which contains neurons that can activate PBN^{CGRP} neurons to promote anorexia (54). Alternatively, it is possible that unilateral CCK^{VN→PBN} activation was not sufficient to recruit a large population of CGRP^{PBN} neurons.

Thus, our data show that alterations in the activity of CCK^{VN} neurons exert a pivotal role in controlling MS-like behavioral responses. Here we provide several lines of evidence that CCK signaling, through its CCK-A receptor, underlies MS responses. Devazepide administration, a CCK-A receptor antagonist, abolishes both hypolocomotion and CTA after a rotational stimulus, without affecting spontaneous locomotion like dimenhydrinate, a widely used anti-motion sickness drug.

Pharmacologically, three anti-MS drug classes have been described depending on their influence over MS habituation. Thus, class A drugs such as amphetamine are thought to block the MS-eliciting sensory input, leading to habituation delay. Class B drugs such as anticholinergics modulate the neural store decreasing the neuronal mismatch signal intensity, leading to boosted habituation. On the other hand, class C drugs such as antihistamines inhibit MS-related autonomic responses, leading to unchanged MS habituation (60). Even though we have not tested habituation *per se*, our results suggest that devazepide may be preventing the expression of autonomic responses, akin to class C drugs. Mechanistically, we show that CCK-A receptor blockade reduces CGRP^{PBN} activation, in agreement with our recent study reporting the expression of *Cckra* in CGRP^{PBN} neurons (48). In addition to its known gastric effects, CCK-A receptor signaling has been suggested to contribute to multiple central functions, such as anxiety, nociception, and food consumption (6). In this regard, CCK administration activates CGRP^{PBN} neurons, which are known to both elicit CTA and retain CTA-related aversive memories by conveying the required gastrointestinal distress input (15, 17).

Our study underscores a key role for CCK^{VN} neurons in MS-related behavioral and physiologic responses by impinging onto PBN circuitry, providing the first evidence of a direct link between motion inputs and aversive responses.

### REFERENCES

(1) Abe, C., Tanaka, K., Iwata, C., & Morita, H. (2010). Vestibular-mediated increase in central serotonin plays an important role in hypergravity-induced hypophagia in rats. Journal of Applied Physiology, 109(6), 1635-1643. https://doi.org/10.1152/japplphysiol.00515.2010
(2) Abe, C., Yamaoka, Y., Maejima, Y., Mikami, T., Yokota, S., Yamanaka, A., & Morita, H. (2020). VGLUT2-expressing neurons in the vestibular nuclear complex mediate gravitational stress-induced hypothermia in mice. Commun Biol, 3(1), 227. https://doi.org/10.1038/s42003-020-0950-0
(3) Angelaki, D. E., & Cullen, K. E. (2008). Vestibular system: the many facets of a multimodal sense. Annu Rev Neurosci, 31, 125-150. https://doi.org/10.1146/annurev.neuro.31.060407.125555
(4) Balaban, C. D. (1996). Vestibular nucleus projections to the parabrachial nucleus in rabbits: implications for vestibular influences on the autonomic nervous system. Exp Brain Res, 108(3), 367-381. http://www.ncbi.nlm.nih.gov/pubmed/8801117
(5) Balaban, C. D., & Beryozkin, G. (1994). Vestibular nucleus projections to nucleus tractus solitarius and the dorsal motor nucleus of the vagus nerve: potential substrates for vestibulo-autonomic interactions. Experimental brain research, 98(2), 200-212. https://doi.org/10.1007/bf00228409
(6) Ballaz, S. (2017). The unappreciated roles of the cholecystokinin receptor CCK(1) in brain functioning. Rev Neurosci, 28(6), 573-585. https://doi.org/10.1515/revneuro-2016-0088
(7)Bankhead, P., Loughrey, M. B., Fernandez, J. A., Dombrowski, Y., McArt, D. G., Dunne, P. D., McQuaid, S., Gray, R. T., Murray, L. J., Coleman, H. G., James, J. A., Salto-Tellez, M., & Hamilton, P. W. (2017). QuPath: Open source software for digital pathology image analysis. Sci Rep, 7(1), 16878. https://doi.org/10.1038/s41598-017-17204-5
(8) Bernstein, I. L. (1978). Learned taste aversions in children receiving chemotherapy. Science, 200(4347), 1302-1303. https://doi.org/10.1126/science.663613
(9) Bertolini, G., & Straumann, D. (2016). Moving in a Moving World: A Review on Vestibular Motion Sickness. Frontiers in neurology, 7, 14-14. https://doi.org/10.3389/fneur.2016.00014
(10) Bles, W., Bos, J. E., de Graaf, B., Groen, E., & Wertheim, A. H. (1998). Motion sickness: only one provocative conflict? Brain Res Bull, 47(5), 481-487. https://doi.org/10.1016/s0361-9230(98)00115-4
(11) Bolea, I., Gella, A., Sanz, E., Prada-Dacasa, P., Menardy, F., Bard, A. M., Machuca-Marquez, P., Eraso-Pichot, A., Modol-Caballero, G., Navarro, X., Kalume, F., & Quintana, A. (2019). Defined neuronal populations drive fatal phenotype in a mouse model of Leigh syndrome. Elife, 8. https://doi.org/10.7554/eLife.47163
(12) Borgius, L., Restrepo, C. E., Leao, R. N., Saleh, N., & Kiehn, O. (2010). A transgenic mouse line for molecular genetic analysis of excitatory glutamatergic neurons. Mol Cell Neurosci, 45(3), 245-257. https://doi.org/10.1016/j.mcn.2010.06.016
(13) Braun, J. J., & Mclntosh, H. (1973). Learned taste aversions induced by rotational stimulation. Physiological Psychology, 1(4), 301-304. https://doi.org/10.3758/BF03326928
(14) Cai, Y.-L., Ma, W.-L., Li, M., Guo, J.-S., Li, Y.-Q., Wang, L.-G., & Wang, W.-Z. (2007). Glutamatergic vestibular neurons express Fos after vestibular stimulation and project to the NTS and the PBN in rats. Neuroscience Letters, 417(2), 132-137. https://doi.org/10.1016/j.neulet.2007.01.079
(15) Carter, M. E., Han, S., & Palmiter, R. D. (2015). Parabrachial calcitonin gene-related peptide neurons mediate conditioned taste aversion. J Neurosci, 35(11), 4582-4586. https://doi.org/10.1523/JNEURQSCI.3729-14.2015
(16) Carter, M. E., Soden, M. E., Zweifel, L. S., & Palmiter, R. D. (2013). Genetic identification of a neural circuit that suppresses appetite. Nature, 503(7474), 111-114. https://doi.org/10.1038/nature12596
(17) Chen, J. Y., Campos, C. A., Jarvie, B. C., & Palmiter, R. D. (2018). Parabrachial CGRP Neurons Establish and Sustain Aversive Taste Memories. Neuron, 100(4), 891-899 e895. https://doi.org/10.1016/j.neuron.2018.09.032
(18) Dieterich, M., Bense, S., Stephan, T., Brandt, T., Schwaiger, M., & Bartenstein, P. (2005). Medial Vestibular Nucleus Lesions in Wallenberg's Syndrome Cause Decreased Activity of the Contralateral Vestibular Cortex. Annals of the New York Academy of Sciences, 1039(1), 368-383. https://doi.org/10.1196/annals.1325.035
(19) Erö, C., Gewaltig, M.-O., Keller, D., & Markram, H. (2018). A Cell Atlas for the Mouse Brain. Frontiers in Neuroinformatics, 12, 84-84. https://doi.org/10.3389/fninf.2018.00084
(20)Franklin, K. B. J., & Paxinos, G. (2013). Paxinos and Franklin's The mouse brain in stereotaxic coordinates (Fourth edition. ed.). Academic Press, an imprint of Elsevier.
(21) Fuller, P. M., Jones, T. A., Jones, S. M., & Fuller, C. A. (2002). Neurovestibular modulation of circadian and homeostatic regulation: vestibulohypothalamic connection? Proceedings of the National Academy of Sciences of the United States of America, 99(24), 15723-15728. https://doi.org/10.1073/pnas.242251499
(22) Gagliuso, A. H., Chapman, E. K., Martinelli, G. P., & Holstein, G. R. (2019). Vestibular neurons with direct projections to the solitary nucleus in the rat. J Neurophysiol, 122(2), 512-524. https://doi.org/10.1152/jn.00082.2019
(23) Graybiel, A., Wood, C. D., Miller, E. F., & Cramer, D. B. (1968). Diagnostic criteria for grading the severity of acute motion sickness. Aerospace medicine, 39(5), 453-455. http://www.ncbi.nlm.nih.gov/pubmed/5648730
(24) Helling, K., Hausmann, S., Clarke, A., & Scherer, H. (2003). Experimentally induced motion sickness in fish: possible role of the otolith organs. Acta otolaryngologica, 123(4), 488-492. https://doi.org/10.1080/0036554021000028121
(25) Highstein, S. M., & Holstein, G. R. (2006). The anatomy of the vestibular nuclei. Prog Brain Res, 151, 157-203. https://doi.org/10.1016/S0079-6123(05)51006-9
(26) Holstein, G. R., Friedrich, V. L., Jr., & Martinelli, G. P. (2016). Glutamate and GABA in Vestibulo-Sympathetic Pathway Neurons. Front Neuroanat, 10, 7. https://doi.org/10.3389/fnana.2016.00007
(27) Horowitz, S. S., Blanchard, J., & Morin, L. P. (2005). Medial vestibular connections with the hypocretin (orexin) system. J Comp Neurol, 487(2), 127-146. https://doi.org/10.1002/cne.20521
(28) Irwin, J. A. (1881). The pathology of sea-sickness. The Lancet, 118(3039), 907-909. https://doi.org/10.1016/S0140-6736(02)38129-7
(29) James, W. (1883). THE SENSE OF DIZZINESS IN DEAF-MUTES. American Annals of the Deaf and Dumb, 28(2), 102-117. http://www.jstor.org/stable/44460811
(30) Kodama, T., Guerrero, S., Shin, M., Moghadam, S., Faulstich, M., & du Lac, S. (2012). Neuronal classification and marker gene identification via single-cell expression profiling of brainstem vestibular neurons subserving cerebellar learning. J Neurosci, 32(23), 7819-7831. https://doi.org/10.1523/JNEUROSCI.0543-12.2012 32/23/7819 [pii]
(31) Lackner, J. R. (2014). Motion sickness: more than nausea and vomiting. Exp Brain Res, 232(8), 2493-2510. https://doi.org/10.1007/s00221-014-4008-8
(32) Lidvall, H. F. (1962). Mechanisms of motion sickness as reflected in the vertigo and nystagmus responses to repeated caloric stimuli. Acta Otolaryngol, 55, 527-536. https://doi.org/10.3109/00016486209127388
(33) Lin, Y., & Carpenter, D. O. (1993). Medial vestibular neurons are endogenous pacemakers whose discharge is modulated by neurotransmitters. Cellular and Molecular Neurobiology, 13(6), 601-613. https://doi.org/10.1007/BF00711560
(34) Liu, S., Ye, M., Pao, G. M., Song, S. M., Jhang, J., Jiang, H., Kim, J. H., Kang, S. J., Kim, D. I., & Han, S. (2022). Divergent brainstem opioidergic pathways that coordinate breathing with pain and emotions. Neuron, 110(5), 857-873 e859. https://doi.org/10.1016/i.neuron.2021.11.029
(35) Mano, N., Oshima, T., & Shimazu, H. (1968). Inhibitory commissural fibers interconnecting the bilateral vestibular nuclei. Brain Res, 8(2), 378-382. https://doi.org/10.1016/0006-8993(68)90058-9
(36) Money, K. E. (1970). Motion sickness. Physiological Reviews, 50(1), 1-39. http://www.ncbi.nlm.nih.gov/pubmed/4904269
(37) Montardy, Q., Wei, M., Liu, X., Yi, T., Zhou, Z., Lai, J., Zhao, B., Besnard, S., Tighilet, B., Chabbert, C., & Wang, L. (2021). Selective optogenetic stimulation of glutamatergic, but not GABAergic, vestibular nuclei neurons induces immediate and reversible postural imbalance in mice. Prog Neurobiol, 204, 102085. https://doi.org/10.1016/i.pneurobio.2021.102085
(38) Murakami, D. M., Erkman, L., Hermanson, O., Rosenfeld, M. G., & Fuller, C. A. (2002). Evidence for vestibular regulation of autonomic functions in a mouse genetic model. Proceedings of the National Academy of Sciences, 99(26), 17078-17082. https://doi.org/10.1073/PNAS.252652299
(39) Nalivaiko, E. (2018). Thermoregulation and nausea. In (Vol. 156, pp. 445-456). https://doi.org/10.1016/B978-0-444-63912-7.00027-8
(40) Nalivaiko, E., Rudd, J. A., & So, R. H. Y. (2014). Motion sickness, nausea and thermoregulation: The "toxic" hypothesis. Temperature: Multidisciplinary Biomedical Journal, 1(3), 164-164. https://doi.org/10.4161/23328940.2014.982047
(41) Ng, L., Bernard, A., Lau, C., Overly, C. C., Dong, H.-W., Kuan, C., Pathak, S., Sunkin, S. M., Dang, C., Bohland, J. W., Bokil, H., Mitra, P. P., Puelles, L., Hohmann, J., Anderson, D. J., Lein, E. S., Jones, A. R., & Hawrylycz, M. (2009). An anatomic gene expression atlas of the adult mouse brain. Nature Neuroscience, 12(3), 356-362. https://doi.org/10.1038/nn.2281
(42) Ngampramuan, S., Cerri, M., Del Vecchio, F., Corrigan, J. J., Kamphee, A., Dragic, A. S., Rudd, J. A., Romanovsky, A. A., & Nalivaiko, E. (2014). Thermoregulatory correlates of nausea in rats and musk shrews. Oncotarget, 5(6), 1565-1575. https://doi.org/10.18632/oncotarget.1732
(43)Nijsen, M. J., Croiset, G., Stam, R., Bruijnzeel, A., Diamant, M., de Wied, D., & Wiegant, V. M. (2000). The role of the CRH type 1 receptor in autonomic responses to corticotropin- releasing hormone in the rat. Neuropsychopharmacology, 22(4), 388-399. https://doi.org/10.1016/S0893-133X(99)00126-8
(44) Norris, A. J., Shaker, J. R., Cone, A. L., Ndiokho, I. B., & Bruchas, M. R. (2021). Parabrachial opioidergic projections to preoptic hypothalamus mediate behavioral and physiological thermal defenses. Elife, 10. https://doi.org/10.7554/eLife.60779
(45) Oman, C. M. (1991). Sensory conflict in motion sickness: an Observer Theory approach. Pictorial communication in virtual and real environments, 362-376. https://ci.nii.ac.ip/naid/10007443135
(46) Palmiter, R. D. (2018). The Parabrachial Nucleus: CGRP Neurons Function as a General Alarm. Trends in neurosciences, 41(5), 280-293. https://doi.org/10.1016/j.tins.2018.03.007
(47) Parrott, R. F., Ebenezer, I. S., Baldwin, B. A., & Forsling, M. L. (1991). Central and peripheral doses of cholecystokinin that inhibit feeding in pigs also stimulate vasopressin and cortisol release. Exp Physiol, 76(4), 525-531. https://doi.org/10.1113/expphysiol.1991.sp003518
(48) Pauli, J. L., Chen, J. Y., Basiri, M. L., Park, S., Carter, M. E., Sanz, E., McKnight, G. S., Stuber, G. D., & Palmiter, R. D. (2022). Molecular and anatomical characterization of parabrachial neurons and their axonal projections. Elife, 11. https://doi.org/10.7554/eLife.81868
(49) Previc, F. H. (1993). Do the organs of the labyrinth differentially influence the sympathetic and parasympathetic systems? Neuroscience and biobehavioral reviews, 17(4), 397-404. https://doi.org/10.1016/s0149-7634(05)80116-2
(50) Previc, F. H. (2018). Intravestibular Balance and Motion Sickness. Aerosp Med Hum Perform, 89(2), 130-140. https://doi.org/10.3357/AMHP.4946.2018
(51) Quintana, A., Zanella, S., Koch, H., Kruse, S. E., Lee, D., Ramirez, J. M., & Palmiter, R. D. (2012). Fatal breathing dysfunction in a mouse model of Leigh syndrome. J Clin Invest, 122(7), 2359-2368. https://doi.org/10.1172/JCI62923
(52) Reason, J. T. (1978). Motion Sickness Adaptation: A Neural Mismatch Model. Journal of the Royal Society of Medicine, 71(11), 819-829. https://doi.org/10.1177/014107687807101109
(53) Ris, L., Capron, B., Vibert, N., Vidal, P. P., & Godaux, E. (2001). Modification of the pacemaker activity of vestibular neurons in brainstem slices during vestibular compensation in the guinea pig. Eur J Neurosci, 13(12), 2234-2240. https://doi.org/10.1046/i.0953-816x.2001.01603.x
(54) Roman, C. W., Derkach, V. A., & Palmiter, R. D. (2016). Genetically and functionally defined NTS to PBN brain circuits mediating anorexia. Nat Commun, 7, 11905. https://doi.org/10.1038/ncomms11905
(55) Sanz, E., Bean, J. C., Carey, D. P., Quintana, A., & McKnight, G. S. (2019). RiboTag: Ribosomal Tagging Strategy to Analyze Cell-Type-Specific mRNA Expression In Vivo. Curr Protoc Neurosci, 88(1), e77. https://doi.org/10.1002/cpns.77
(56) Sanz, E., Quintana, A., Deem, J. D., Steiner, R. A., Palmiter, R. D., & McKnight, G. S. (2015). Fertility-regulating Kiss1 neurons arise from hypothalamic POMC-expressing progenitors. J Neurosci, 35(14), 5549-5556. https://doi.org/10.1523/JNEUROSCI.3614-14.201535/14/5549 [pii]
(57) Sanz, E., Yang, L., Su, T., Morris, D. R., McKnight, G. S., & Amieux, P. S. (2009). Cell-type-specific isolation of ribosome-associated mRNA from complex tissues. Proc Natl Acad Sci U S A, 106(33), 13939-13944. http://www.ncbi.nlm.nih.gov/entrez/query.fcgi?cmd=Retrieve&db=PubMed&dopt=Citati on&list uids=19666516
(58) Shi, X., Wei, H., Chen, Z., Wang, J., Qu, W., Huang, Z., & Dai, C. (2021). Whole-brain monosynaptic inputs and outputs of glutamatergic neurons of the vestibular nuclei complex in mice. HearRes, 401, 108159. https://doi.org/10.1016/j.heares.2020.108159
(59) Singh, P., Yoon, S. S., & Kuo, B. (2016). Nausea: a review of pathophysiology and therapeutics. Therap Adv Gastroenterol, 9(1), 98-112. https://doi.org/10.1177/1756283X15618131
(60)Takeda, N., Morita, M., Horii, A., Nishiike, S., Kitahara, T., & Uno, A. (2001). Neural mechanisms of motion sickness. J Med Invest, 48(1-2), 44-59. https://www.ncbi.nlm.nih.gov/pubmed/11286016
(61) Tan, C. L., Cooke, E. K., Leib, D. E., Lin, Y. C., Daly, G. E., Zimmerman, C. A., & Knight, Z. A. (2016). Warm-Sensitive Neurons that Control Body Temperature. Cell, 167(1), 47-59 e15. https://doi.org/10.1016/j.cell.2016.08.028
(62) Taniguchi, H., He, M., Wu, P., Kim, S., Paik, R., Sugino, K., Kvitsiani, D., Fu, Y., Lu, J., Lin, Y., Miyoshi, G., Shima, Y., Fishell, G., Nelson, S. B., & Huang, Z. J. (2011). A resource of Cre driver lines for genetic targeting of GABAergic neurons in cerebral cortex. Neuron, 71(6), 995-1013. https://doi.org/10.1016/j.neuron.2011.07.026
(63) Treisman, M. (1977). Motion sickness: an evolutionary hypothesis. Science, 197(4302), 493-495. https://doi.org/10.1126/science.301659
(64) Waespe, W., & Henn, V. (1979). The velocity response of vestibular nucleus neurons during vestibular, visual, and combined angular acceleration. Exp Brain Res, 37(2), 337-347. https://doi.org/10.1007/BF00237718
(65) Wei, X., Wang, Z.-B., Zhang, L.-C., Liu, W.-Y., Su, D.-F., & Li, L. (2011). Verification of motion sickness index in mice. CNS neuroscience & therapeutics, 17(6), 790-792. https://doi.org/10.1111/j.1755-5949.2011.00272.x
(66) Xie, Z., Zhang, X., Zhao, M., Huo, L., Huang, M., Li, D., Zhang, S., Cheng, X., Gu, H., Zhang, C., Zhan, C., Wang, F., Shang, C., & Cao, P. (2022). The gut-to-brain axis for toxin-induced defensive responses. Cell, 185(23), 4298-4316 e4221. https://doi.org/10.1016/j.cell.2022.10.001
(67) Yates, B. J., Holmes, M. J., & Jian, B. J. (2000). Adaptive plasticity in vestibular influences on cardiovascular control. Brain research bulletin, 53(1), 3-9. https://doi.org/10.1016/s0361-9230(00)00302-6
(68) Yates, B. J., & Miller, A. D. (1994). Properties of sympathetic reflexes elicited by natural vestibular stimulation: implications for cardiovascular control. Journal of Neurophysiology, 71(6), 2087-2092. https://doi.org/10.1152/jn.1994.71.6.2087
(69) Zhang, Z. H., Liu, L. P., Fang, Y., Wang, X. C., Wang, W., Chan, Y. S., Wang, L., Li, H., Li, Y. Q., & Zhang, F. X. (2022). A New Vestibular Stimulation Mode for Motion Sickness With Emphatic Analysis of Pica. Front Behav Neurosci, 16, 882695. https://doi.org/10.3389/fnbeh.2022.882695

## Claims

1. A cholecystokinin (CCK) antagonist for use in the prevention and/or treatment of a vestibular disease or disorder selected from motion sickness, Meniere's disease, vestibular migraine, disembarkment sickness, labyrinthitis, vestibular neuronitis, benign paroxysmal positional vertigo, labyrinthine infarction and nausea.

2. The CCK antagonist for use according to claim 1, wherein said CCK antagonist is selected from a benzodiazepine, benzodiazepinone, and a combination thereof.

3. The CCK antagonist for use according to claim 1, wherein said CCK antagonist is selected from dexloxiglumide, devazepide, TS-941 ((S)-(-)-N-[2,3-dihydro-2-oxo-5-phenyl-1-[(1H-tetrazol-5-yl)methyl]-1H-1,4-benzodiazepine-3-yl]-2-indolecarboxamide), CCK-OPE (_{D}Tyr-Gly-Asp-Tyr(SO₃H)-Nle-Gly-Trp-Nle-Asp-O-phenylethyl ester), JMV179 ((6S,9S,15R,18S,21S)-15-((1H-indol-3-yl)methyl)-9,18-dibutyl-2,2-dimethyl-4,7,10,13,16,19-hexaoxo-21-(phenethoxycarbonyl)-6-(4-(sulfooxy)benzyl)-3-oxa-5,8,11,14,17,20-hexaazatricosan-23-oic acid), Lintitript (2-[2-[[4-(2-chlorophenyl)-1,3-thiazol-2-yl]carbamoyl]indol-1-yl]acetic acid), IQM-95,333 (2-benzyl-5-(N-((tert-butoxycarbonyl)-L-tryptophyl)amino)-1,3-dioxoperhydropyrido(1,2-c)pyrimidine), KSG-504 (4(R)-[N-(3-Methoxypropyl)-N-Pentylcarbamoyl]-5-(2-Naphthylsulfonyl)Pentanoic Acid L-Arginine Salt Monohydrate), T-0632 ([sodium (S)-3-[1-(2-fluorophenyl)-2,3-dihydro-3-[(3-isoquinolinyl)-carbonyl] amino-6-methoxy-2-oxo-1-H-indole]propanoate]) and TP680 ((R)-1-[3-(3-carboxypyridine-2-yl) thio-2-(indol-2-yl)carbonylamino]propionyl-4-diphenylmethyl- piperazine), and a combination thereof.

4. The CCK antagonist for use according to claim 3, wherein said CCK antagonist is devazepide.

5. The CCK antagonist for use according to any of claims 1 to 4, wherein the vestibular disease or disorder is motion sickness.

6. The CCK antagonist for use according to any of the preceding claims, wherein said CCK antagonist is combined with at least one another active ingredient.

7. The CCK antagonist for use according to claim 6, wherein said another active ingredient is a compound selected from an anti-histamine, anti-cholinergic, anti-dopaminergic drug, sympathomimetic and benzodiazepine.

8. The CCK antagonist for use according to claim 7, wherein said anti-histamine compound is selected from dimenhydrinate, meclizine and promethazine.

9. The CCK antagonist for use according to claim 7, wherein said anti-cholinergic compound is scopolamine.

10. The CCK antagonist for use according to claim 7, wherein said anti-dopaminergic drug is selected from prochlorperazine and metoclopramide.

11. The CCK antagonist for use according to any of the preceding claims, wherein said CCK antagonist is included in a pharmaceutical composition.

12. The CCK antagonist for use according to claim 11, wherein said pharmaceutical composition further comprises a pharmaceutically acceptable vehicle, adjuvant, diluent or excipient, and optionally another active ingredient.

13. The CCK antagonist for use according to any of the preceding claims, wherein said prevention or treatment comprises orally, intravenously or intraperitoneally administering said CCK antagonist or the pharmaceutical composition comprising thereof.

14. The CCK antagonist for use according to claim 13, wherein said prevention or treatment comprises orally administering said CCK antagonist or the pharmaceutical composition comprising thereof.

15. The CCK antagonist for use according to any of claims 1 to 10 or 14, wherein said CCK antagonist is included in a functional food or a dietary supplement, optionally combined with at least one another active ingredient,.
